(19) Europäisches Patentamt
European Patent Office
Office européen des brevets

(11) Veröffentlichungsnummer: **0 173 933 B1**

(12) # EUROPÄISCHE PATENTSCHRIFT

(45) Veröffentlichungstag der Patentschrift: **29.11.89**

(51) Int. Cl.⁴: **C 07 D 471/04**, A 61 K 31/435 // C07D213/80 ,(C07D471/04, 221:00, 221:00)

(21) Anmeldenummer: **85110646.8**

(22) Anmeldetag: **23.08.85**

(54) **1,6-Naphthyridin-Derivate, Verfahren zu deren Herstellung und diese enthaltende Arzneimittel.**

(30) Priorität: **25.08.84 DE 3431303**
**29.01.85 DE 3502790**

(43) Veröffentlichungstag der Anmeldung: **12.03.86 Patentblatt 86/11**

(45) Bekanntmachung des Hinweises auf die Patenterteilung: **29.11.89 Patentblatt 89/48**

(84) Benannte Vertragsstaaten: **AT BE CH DE FR GB IT LI LU NL SE**

(56) Entgegenhaltungen:
**EP-A- 0 133 530**
**US-A- 4 304 914**

**Die Akte enthält technische Angaben, die nach dem Eingang der Anmeldung eingereicht wurden und die nicht in dieser Patentschrift enthalten sind.**

(73) Patentinhaber: **GÖDECKE AKTIENGESELLSCHAFT, Salzufer 16, D-1000 Berlin 10 (DE)**

(72) Erfinder: **Satzinger, Gerhard, Dr., Im Mattenbühl 7, D-7809 Denzlingen (DE)**
Erfinder: **Hartenstein, Johannes, Dr., Fohrenbühl 23, D-7801 Stegen-Wittental (DE)**
Erfinder: **Mannhardt, Karl, Dr., Pfauenstrasse 14, D-7807 Elzach-Oberprechtal (DE)**
Erfinder: **Kleinschroth, Jürgen, Dr., Freiburger Strasse 13, D-7809 Denzlingen (DE)**
Erfinder: **Osswald, Hartmut, Prof. Dr., Kiefernweg 1, D-7808 Waldkirch 2 (DE)**
Erfinder: **Weinheimer, Günter, Dr., Sachsenstrasse 4, D-7809 Denzlingen (DE)**
Erfinder: **Fritschi, Edgar, Dr., Am Scheuerwald 2, D-7811 St. Peter (DE)**

**EP 0 173 933 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 1,6-Naphthyridin-Derivate der allgemeinen Formel I

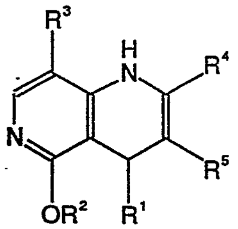

(I)

in welcher
$R^1$ einen unsubstituierten oder substituierten aromatischen oder heteroaromatischen Ring,
$R^2$ eine geradkettige oder verzweigte Alkylgruppe mit bis zu 4 Kohlenstoffatomen oder eine Benzylgruppe,
$R^3$ Wasserstoff, eine geradkettige oder verzweigte Alkylgruppe oder einen Alkoxycarbonylrest mit bis zu 5 Kohlenstoffatomen
$R^4$ eine geradkettige oder verzweigte Alkylgruppe mit bis zu 4 Kohlenstoffatomen und
$R^5$ eine Carboxylgruppe oder einen geradkettigen, verzweigten oder cyclischen Alkoxycarbonylrest, der bis zu 17 Kohlenstoffatome und gegebenenfalls auch ein Sauerstoff-, Schwefel- oder Stickstoffatom enthält, bedeuten
sowie gegebenenfalls deren pharmakologisch unbedenkliche Salze.

Als aromatische oder heteroaromatische Ringe $R^1$ kommen vor allem infrage unsubstituierte oder ein- oder zweifach durch niedrige polare oder unpolare Reste substituierte Phenyl-, Thienyl-, Pyridyl- oder 2,1,3-Benzoxadiazolylreste.

Es werden solche 1,6-Naphthyridinderivate bevorzugt, in welchen
$R^1$ einen unsubstituierten oder durch Halogen, Cyano, Nitro, Niederalkyl, Niederalkoxy, Difluormethoxy, Trifluormethoxy, Niederalkylendioxy wie z. B. Methylendioxy, Niederalkylamino insbesondere Dimethyl- oder Diethylamino, Methylthio- oder Trifluormethylreste mono- oder disubstituierten Phenylrest oder einen Thienyl-, Pyridyl-, oder 2,1,3-Benzoxadiazolylrest bedeutet.
$R^3$ ist bevorzugt Wasserstoff, 1-4 C Alkyl oder eine niedere Alkoxycarbonylgruppe mit bis zu 5 Kohlenstoffatomen
$R^4$ bedeutet bevorzugt eine niedere Alkylgruppe mit 1–3 Kohlenstoffatomen, insbesondere die Methyl- oder Ethylgruppe
$R^5$ stellt bevorzugt eine Carboxyl- oder Alkoxycarbonylgruppe mit bis zu 17 C-Atomen dar. Es können hier auch kompliziertere und voluminösere Reste infrage kommen, die gegebenenfalls weitere Heteroatome, wie Sauerstoff-, Schwefel- oder Stickstoffatome enthalten. Typische Beispiele solcher Reste sind Amine, wie niedere N-Benzyl-N-alkylaminoalkylreste, N,N-Dialkylaminoalkylreste oder niedere Alkylthioalkyl- oder Alkoxyalkylreste.

Besonders bevorzugt werden 1,6-Naphthyridin-Derivate der allgemeinen Formel I, in welcher
$R^1$ einen unsubstituierten oder einen vorzugsweise in 2- oder 3-Position durch Halogen, Cyano, Nitro, Methyl, Methoxy, Difluormethoxy oder Trifluormethyl substituierten Phenylrest oder einen vorzugsweise in 2,3- oder 2,6-Position durch Halogenatome, die gleich oder verschieden sein können, disubstituierten Phenylrest oder einen unsubstituierten Thienylrest
$R^2$ einen Methyl-, Ethyl-, n-Propyl-, Isopropyl-, n-Butyl-, sek. Butyl-, Isobutyl- oder Benzylrest
$R^3$ Wasserstoff, vorrangig die Methyl- aber auch die Ethyl- oder Isopropylgruppe oder einen Alkoxycarbonylrest der allgemeinen Formel II

$$-CO_2R^6 \qquad (II)$$

in welcher $R^6$ eine Methyl-, Ethyl-, Propyl- oder Isopropylgruppe insbesondere die Ethylgruppe bedeutet
$R^4$ einen Methyl- oder Ethylrest und
$R^5$ einen Carboxyl- oder Alkoxycarbonylrest der allgemeinen Formel III

$$-CO_2R^7 \qquad (III)$$

in welcher $R^7$ entweder Wasserstoff, einen Methyl-, Ethyl-, Propyl-Isopropyl-, n-Butyl-, Isobutyl-, tert. Butyl- oder Benzylrest, eine Alkoxyalkyl- oder Alkylthioalkylgruppe der allgemeinen Formeln IV und V

$$-(CH_2)_n-O-R^8 \qquad (IV)$$
$$-(CH_2)_n-S-R^8 \qquad (V)$$

worin $R^8$ eine niedere Alkylgruppe mit 1–3 Kohlenstoffatomen und n die Zahlen 2 oder 3 bedeutet oder eine Aminoalkylgruppe der allgemeinen Formel VI

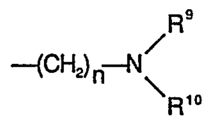

(VI)

worin $R^9$ und $R^{10}$ gleich oder verschieden sein können und Wasserstoff eine geradkettige oder verzweigte niedere Alkylgruppe mit 1–4 Kohlenstoffatomen oder eine Benzylgruppe darstellen oder gemeinsam eine niedere Alkylengruppe bilden und n die Zahlen 2 oder 3 bedeutet, darstellt.

Die Erfindung betrifft weiterhin ein Verfahren zur Herstellung von 1,6-Naphthyridin-Derivaten der allgemeinen Formel I, dadurch gekennzeichnet, dass man 1,6-Naphthyridinon-Derivate der allgemeinen Formel VII

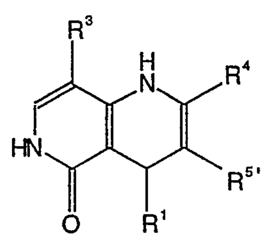

(VII)

in welcher $R^1$, $R^3$ und $R^4$ die oben angegebenen Bedeutung haben und $R^{5'}$ einen Alkoxycarbonylrest der allgemeinen Formel III bedeutet, in an sich bekannter Weise alkyliert.

Verbindungen der allgemeinen Formel I, bei denen $R^5$ für eine Carboxylgruppe steht, werden bevorzugt hergestellt, indem man Verbindungen der allgemeinen Formel I, in denen $R^5$ für einen Benzyloxycarbonylrest steht, in an sich bekannter Weise hydrogenolytisch spaltet.

Die Verbindungen der allgemeinen Formel VII können z.B. nach der deutschen Patentanmeldung DE-A 3 327 650 hergestellt werden, indem man entweder

a) ein Dihydropyridin der allgemeinen Formel VIII

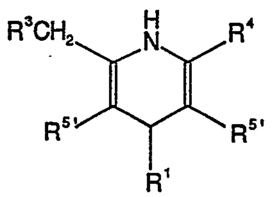

(VIII)

in welcher $R^1$, $R^3$, $R^4$ und $R^{5'}$ die oben genannte Bedeutung haben, in Gegenwart einer Base mit s-Triazin umsetzt,

b) ein 1,4-Dihydropyridin der allgemeinen Formel VIII mit einem Dialkylformamid-dialkylacetal der allgemeinen Formel IX

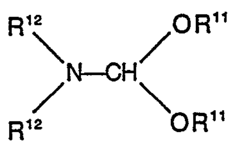

(IX)

in welcher $R^{12}$ gleich oder verschieden sein kann und eine Methyl- oder Ethylgruppe bedeutet und die Reste $R^{11}$ jeweils eine Alkylgruppe mit bis zu 4 Kohlenstoffatomen oder gemeinsam eine Alkylengruppe mit bis zu 3 Kohlenstoffatomen bedeuten,

zur Reaktion bringt und die erhaltenen Verbindungen der allgemeinen Formel X

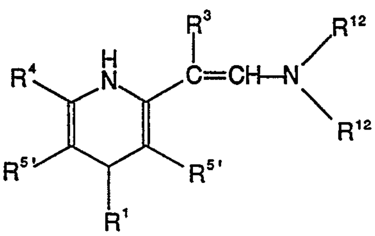

(X)

in welcher $R^1$, $R^3$, $R^4$, $R^{5'}$ und $R^{12}$ die oben angegebene Bedeutung besitzen, mit Ammoniak umsetzt, oder,

c) 2,4-Dihydroxypyridin mit einer Verbindung der allgemeinen Formel XI

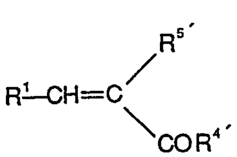

(XI)

in welcher $R^1$ und $R^{5'}$ die oben genannte Bedeutung haben, und $R^{4'}$ eine geradkettige oder verzweigte Alkylgruppe mit bis zu 4 Kohlenstoffatomen bedeutet, in Gegenwart von Ammoniak umsetzt.

Die Verbindungen der allgemeinen Formel XI sind bekannt oder können nach literaturbekannten Verfahren hergestellt werden [Org. Reactions, Vol. 15 (1967) S. 204 ff.]. 2,4-Dihydroxypyridin ist Handelsprodukt.

Die für das Verfahren a) und b) eingesetzten 1,4-Dihydropyridine der allgemeinen Formel VIII sind bekannt [vgl. z.B. Chem. Rev. 82 (1982) S. 223] oder sie können in analoger Weise hergestellt werden.

Zur Durchführung der Reaktion a) wird das 1,4-Dihydropyridinderivat mit s-Triazin in einem inerten organischen Lösungsmittel in Gegenwart starker Basen wie z.B. Alkalialkoholaten oder Natriumhydrid in einem inerten organischen Lösungsmittel auf Temperaturen von 50–160 °C, vorzugsweise 100–150 °C erhitzt. Als Lösungsmittel eignen sich hier vor allem polare Lösungsmittel wie Dimethylsulfoxid, Dimethylformamid, Ethylenglykoldimethylether oder niedere Alkohole wie Ethanol.

Zur Durchführung der Reaktion nach Verfahrensvariante b) wird das entsprechende 1,4-Dihydropyridinderivat mit einer äquivalenten oder überschüssigen Menge Dialkylformamiddialkylacetal, vorzugsweise in Gegenwart eines aprotischen Lösungsmittels wie Dimethylformamid, Dimethylsulfoxid oder Hexamethylphosphorsäuretriamid, unter Erwärmen umgesetzt. Als geeignete Formamidacetale kommen vor allem Dimethylformamid-dimethylacetal und Dimethylformamid-diethylacetal in Frage.

Das nach der Verfahrensvariante b) erhaltene Zwischenprodukt der allgemeinen Formel X wird durch Reaktion mit Ammoniak in Gegenwart eines vorzugsweise protischen Lösungsmittels bei Raumtemperatur oder bei höherer Temperatur, bevorzugt bei der Siedetemperatur des verwendeten Lösungsmittels, in Verbindungen der allgemeinen Formel VII übergeführt. Als Lösungsmittel sind vor allem niedere Alkohole, wie Methanol oder Ethanol geeignet.

Die Reaktion c) wird vorzugsweise in inerten organischen Lösungsmitteln, insbesondere niederen Alkoholen wie z.B. Methanol, Ethanol oder Isopropanol durchgeführt. Es ist auch zweckmässig, bei höheren Temperaturen, vorzugsweise bei Siedetemperatur des verwendeten Lösungsmittels zu arbeiten. Die Reaktionsprodukte lassen sich mit bekannten Trennverfahren, wie Kristallisation und/oder Chromatographie, isolieren und reinigen.

Die Herstellung der 1,6-Naphthyridin-Derivate der allgemeinen Formel I erfolgt erfindungsgemäss nach üblichen, für die O-Alkylierung von Lactamen in der Literatur beschriebenen Verfahren [vgl. Adv. Heterocyclic Chem. 12 (1970), 185–212]. Geeignete Alkylierungsmittel sind Alkylhalogenide und Alkylsulfonate, Dialkylsulfate und Trialkyloxoniumsalze.

Zur Reaktion mit Alkylhalogeniden werden die Verbindungen der allgemeinen Formel VII in Form ihrer Metallsalze, vorzugsweise ihrer Alkali- oder Silbersalze eingesetzt, die entweder separat hergestellt oder in situ mit Hilfe geeigneter Basen wie Metallhydriden, -carbonaten oder -alkoxiden in einem aprotischen Lösungsmittel erzeugt werden.

Als geeignete Lösungsmittel kommen, in Abhängigkeit vom jeweiligen Alkylierungsmittel, nahezu alle inerten organischen Lösungsmittel in Frage wie offenkettige, cyclische oder auch aromatische Kohlenwasserstoffe z.B. n-Pentan, n-Hexan, Cyclohexan, Benzol oder Toluol, halogenierte Kohlenwasserstoffe, wie Dichlormethan, 1,2-Dichlorethan, Ether wie z.B. Diethylether, 1,2-Dimethoxyethan, sowie dipolare aprotische Lösungsmittel wie Dimethylformamid, Hexamethylphosphorsäuretriamid und Dimethylsulfoxid. Der Temperaturbereich kann je nach verwendetem Lösungsmittel zwischen −20°C und dem Siedepunkt des betreffenden Lösungsmittels variiert werden.

Aufgrund des ambidenten Charakters des Lactamanions erhält man bei der Alkylierung vielfach Gemische aus O- und N-Alkylierungsprodukten je nach Reaktionsbedingungen und verwendetem Alkylierungsmittel [J. Org. Chem. 32 (1967), 4040 ff].

Die Trennung der erhaltenen Produktgemische kann durch chromatographischen Methoden und/oder Kristallisation erfolgen.

Die 1,6-Naphthyridin-Derivate der allgemeinen Formel I, bei denen $R^2$ eine Methyl- oder Ethylgruppe darstellt, werden bevorzugt durch Umsetzung der 1,6-Naphthyridinone der allgemeinen Formel VII mit Trimethyl- bzw. Triethyloxoniumsalzen, insbesondere Trimethyloxoniumtetrafluorborat, in einem aprotischen Lösungsmittel erhalten. Die Herstellung der O-Propyl-, O-Isopropyl-, O-Butyl-, O-sec-Butyl-, O-Isobutyl- und O-Benzylverbindungen erfolgt dagegen vorteilhaft durch Alkylierung der Alkalimetall- oder Silbersalze mit entsprechenden Alkyl- oder Benzylhalogeniden.

Saure oder basische Verbindungen der allgemeinen Formel I, welche für $R^5$ eine Carboxylgruppe bzw. einen substituierten oder unsubstituierten Aminoalkoxycarbonylrest aufweisen, überführt man zum Zwecke der Reinigung und aus galenischen Gründen bevorzugt in kristalline, pharmakologisch verträgliche Salze.

Für den Fall, dass $R^5$ eine Carboxylgruppe darstellt, lassen sich mit Basen, wie z.B. Hydroxiden oder Carbonaten, entsprechende Salze der Alkali- oder Erdalkalimetalle herstellen. Wenn die Reste $R^4$ und/oder $R^5$ basischen Charakter aufweisen, werden Salze in üblicher Weise durch Neutralisation der Basen mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure oder Bernsteinsäure in Frage.

Da die erfindungsgemässen Verbindungen der allgemeinen Formel I am C-4 ein chirales Zentrum aufweisen, können sie entweder als racemische Gemische oder in Form der Enantiomeren vorliegen. Aus der US-A 4 304 914 sind zwar 6,7-diarylsubstituierte Naphthyridon-Derivate bekannt, die als kardiotonisch wirksam bezeichnet werden, die Verbindungen der allgemeinen Formel I der vorliegenden Erfindung sind dahingegen neu und zudem hochwirksame Calciumantagonisten. Im Gegensatz zu anderen bekannten Calciumantagonisten ist ausserdem in therapeutischen Konzentrationen keine Cardiodepression (negativ inotrope, negativ chronotrope Wirkung) zu erwarten. Auch ein calciumagonistischer Effekt (positiv inotrope Wirkung) wie beispielsweise in der EP-A 0 071 819 beschrieben, wurde bei den Verbindungen der vorliegenden Erfindung nicht gefunden.

Aufgrund ihrer gefässspasmolytischen Wirkungen sind sie vor allem bei cerebralen, cardialen und peripheren Gefässerkrankungen wie myokardialer Ischämie, bei cerebralem Infarkt, pulmonalen Thrombosen und bei Arteriosklerose und anderen stenotischen Erscheinungen indiziert, insbesondere weil im Vergleich mit bekannten Präparaten ähnlicher Wirkungsweise negativ inotrope Nebeneffekte weitgehend fehlen. Die 1,6-Naphthyridin-Derivate der vorliegenden Erfindung sind daher wertvolle Mittel für die Bekämpfung der Herz-Kreislauf-Mortalität. Ein weiterer Gegenstand der vorliegenden Erfindung ist daher die Verwendung von 1,6-Naphthyridin-Derivaten der allgemeinen Formel I bei der Bekämpfung von Gefässerkrankungen.

Die erfindungsgemässen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Citrat-Puffer, Ethanol, Komplexbildner (wie Äthylendiamin-tetraessigsäure und deren nichttoxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks- und/oder Süssstoffe enthalten. Die enteral verabreichten Einzeldosen liegen im Bereich von etwa 5 bis 250 mg, vorzugsweise 20–100 mg. Parenteral werden etwa 1 bis 20 mg gegeben.

Die folgenden Beispiele dienen der näheren Erläuterung der Erfindung:

Beispiel 1

(±)-4-(2-Fluorphenyl)-1,4-dihydro-5-isopropoxy-2-methyl-1,6-naphthyridin-3-carbonsäuremethylester

1,1 g (37 mMol) Natriumhydrid (80%ig in Paraffinöl) werden in 70 ml trockenem Dimethylformamid suspendiert und unter Rühren bei Raumtemperatur eine Lösung von 10 g (32 mMol) (±)-4-(2-Fluorphenyl)-1,4,5,6-tetrahydro-2-methyl-5-oxo-1,6-naphthyridin-3-carbonsäuremethylester in 100 ml Dimethylformamid zugetropft. Nach Abklingen der Gasentwicklung wird noch 30 Minuten bei Raumtemperatur nachgerührt. Dann werden 5,9 g (35 mMol) Isopropyljodid in 30 ml Dimethylformamid zugegeben. Man rührt weitere 20 Stunden bei Raumtemperatur, rotiert das Lösungsmittel im Vakuum ab und verrührt den öligen Rückstand mit 100 ml Wasser. Die entstehende hellbraune Kristallmasse wird abgesaugt, mit Wasser gewaschen und getrocknet.

Zur Reinigung des Rohprodukts wird dieses aus einer Mischung von 400 ml Essigsäureethylester und 50 ml Methanol kristallisiert, wobei das O-Alkylierungsprodukt in hoch angereicherter Form als leichterlösliche Komponente in der Mutterlauge verbleibt. Diese wird zunächst über Kieselgel mit Dichlormethan/Methanol 9:1 chromatographiert, wobei das noch vorhandene Ausgangsmaterial vollständig abgetrennt wird; weitere Chromatographie über Kieselgel mit Toluol/Essigsäureethylester 3:1 ergibt nahezu reinen (±)-4-(2-Fluorphenyl)-1,4-dihydro-5-isopropoxy-2-methyl-1,6-naphthyridin-3-carbonsäuremethylester ($R_f$ 0,3). Kristallisation aus n-Hexan/Diisopropylether liefert schliesslich DC-reine Kristalle vom Schmp. 164–165 °C.

Der als Ausgangsprodukt verwendete (±)-4-(2-Fluorphenyl)-1,4,5,6-tetrahydro-2-methyl-5-oxo-1,6-naphthyridin-3-carbonsäuremethylester wird wie folgt hergestellt:

Zu einer Suspension von 3,8 g (130 mMol) Natriumhydrid (80%ig in Paraffinöl) in 60 ml trockenem Dimethylformamid wird unter Stickstoffatmosphäre eine Lösung von 31,5 g (120 mMol) 4-(2-Fluorphenyl)-1,4-dihydro-2,6-dimethylpyridin-3,5-dicarbonsäuredimethylester in 260 ml Dimethylformamid getropft. Bei Nachlassen der Gasentwicklung wird noch 10 Minuten bei Raumtemperatur gerührt und anschliessend werden 10,0 g (120 mMol) s-Triazin in 260 ml Dimethylformamid zugetropft. Die Reaktionsmischung wird 16 Stunden auf 110 °C erhitzt und nach dem Abkühlen im Vakuum eingeengt. Der dunkle Rückstand wird mit 600 ml Aceton verrührt, filtriert und das Filtrat im Vakuum eingeengt. Das Rohprodukt wird mit 300 ml Methanol aufgekocht, die nach dem Abkühlen gebildeten Kristalle abfiltriert und zur weiteren Reinigung aus Methanol umkristallisiert.

Man erhält (±)-4-(2-Fluorphenyl)-1,4,5,6-tetrahydro-2-methyl-5-oxo-1,6-naphthyridin-3-carbonsäuremethylester in Form schwach beiger Kristalle vom Schmp. 315–316 °C (Z).

In analoger Weise werden die folgenden Verbindungen erhalten:

(±)-4-(2-Bromphenyl)-1,4-dihydro-5-isopropoxy-2-methyl-1,6-naphthyridin-3-carbonsäuremethylester (1.a), Schmp. 201–202 °C aus Diisopropylether

(±)-1,4-Dihydro-5-isopropoxy-2-methyl-4-(2-nitrophenyl)-1,6-naphthyridin-3-carbonsäuremethylester (1.b), Schmp. 170 °C aus Diisopropylether/Methanol

(±)-1,4-Dihydro-5-isopropoxy-2-methyl-4-phenyl-1,6-naphthyridin-3-carbonsäuremethylester (1.c), Schmp. 132–133 °C aus n-Hexan

(±)-4-(3-Chlor-2-fluorphenyl)-1,4-dihydro-5-isopropoxy-2-methyl-1,6-naphthyridin-3-carbonsäuremethylester (1.d), Schmp. 166–167 °C aus n-Hexan

(±)-1,4-Dihydro-5-isopropoxy-2-methyl-4-(3-nitrophenyl)-1,6-naphthyridin-3-carbonsäuremethylester (1.e), Schmp. 174–175 °C aus n-Hexan

(±)-1,4-Dihydro-5-isopropoxy-2-methyl-4-(2-trifluormethylphenyl)-1,6-naphthyridin-3-carbonsäuremethylester (1.f), Schmp. 199–200 °C aus Diisopropylether

(±)-4-(2-Chlor-6-fluorphenyl)-1,4-dihydro-5-isopropoxy-2-methyl-1,6-naphthyridin-3-carbonsäuremethylester (1.g), Schmp. 194–195 °C aus Diisopropylether

(±)-1,4-Dihydro-2-methyl-5-propoxy-4-(2-trifluormethylphenyl)-1,6-naphthyridin-3-carbonsäuremethylester (1.h), Schmp. 151–152 °C aus n-Hexan/Diisopropylether

(±)-4-(2-Bromphenyl)-5-ethoxy-1,4-dihydro-2-methyl-1,6-naphthyridin-3-carbonsäuremethylester (1.i), Schmp. 203–204 °C aus Toluol/Ethylacetat

(±)-5-Butoxy-1,4-dihydro-2-methyl-4-(2-trifluormethylphenyl)-1,6-naphthyridin-3-carbonsäureethylester (1.j), Schmp. 107–109 °C aus n-Hexan

(±)-1,4-Dihydro-5-isopropoxy-2-methyl-4-(3-nitrophenyl)-1,6-naphthyridin-3-carbonsäure-(2-methoxyethyl)ester (1.k), Schmp. 174–175 °C aus Diisopropylether

(±)-1,4-Dihydro-5-isopropoxy-2-methyl-4-(2-trifluormethylphenyl)-1,6-naphthyridin-3-carbonsäureethylester (1.l), Schmp. 102–103 °C aus n-Hexan

(±)-1,4-Dihydro-5-isopropoxy-2-methyl-4-(2-trifluormethylphenyl)-1,6-naphthyridin-3-carbonsäureisopropylester (1.m), Schmp. 111–112 °C aus n-Hexan

(±)-1,4-Dihydro-5-isopropoxy-2-methyl-4-(2-trifluormethylphenyl)-1,6-naphthyridin-3-carbonsäureisopropylester (1.n), Schm. 115–116 °C aus n-Hexan

(±)-4-(2,3-Dichlorphenyl)-1,4-dihydro-5-isopropoxy-2-methyl-1,6-naphthyridin-3-carbonsäureethylester (1.o), Schmp. 272–273 °C aus Diisopropylether

(±)-5-Sec-Butoxy-1,4-dihydro-2-methyl-4-(2-trifluormethylphenyl)-1,6-naphthyridin-3-carbonsäureethylester • Hydrochlorid (1.p), Schmp. 148–150 °C aus Diisopropylether/Ethylacetat

(±)-1,4-Dihydro-5-isobutoxy-2-methyl-4-(2-trifluormethylphenyl)-1,6-naphthyridin-3-carbonsäureethylester (1.q), Schmp. 118–119°C aus Petroleumbenzin, 60–80°C

(±)-1,4-Dihydro-5-isopropoxy-2-methyl-4-(2-trifluormethylphenyl)-1,6-naphthyridin-3-carbonsäure-tert.-butylester (1.r), Schmp. 209°C aus n-Hexan/Diisopropylether

(±)-2-Ethyl-1,4-dihydro-5-isopropoxy-8-methyl-4-phenyl-1,6-naphthyridin-3-carbonsäureethylester (1.s), Schmp. 176–177°C aus n-Hexan/Diisopropylether

(±)-1,4-Dihydro-5-isopropoxy-2-methyl-4-(3-nitrophenyl)-1,6-naphthyridin-3-carbonsäure-[2-(N-benzyl-N-methylamino)-ethyl]ester Dihydrochlorid (1.t), Schmp. 148–150°C aus Ethylacetat/Acetonitril

(±)-1,4-Dihydro-5-isopropoxy-2-methyl-4-(3-nitrophenyl)-1,6-naphthyridin-carbonsäure-(2-dimethylaminoethyl)ester Dihydrochlorid (1.u), Schmp. 148–150°C aus Diisopropylether

(±)-1,4-Dihydro-5-isopropoxy-2-methyl-4-(3-nitrophenyl)-1,6-naphthyridin-3-carbonsäure-(2-methylthioethyl)ester (1.v), Schmp. 154–155C aus Diisopropylether/Ethylacetat

(±)-1,4-Dihydro-5-isopropoxy-2-methyl-4-(2-trifluormethylphenyl)-1,6-naphthyridin-3-carbonsäure-[2-(N-benzyl-N-methylamino)ethyl]ester Dihydrochlorid (1.w), Schmp. 163-165°C (Z.) aus Acetonitril

(±)-1,4-Dihydro-5-isopropoxy-2-methyl-4-(2-trifluormethylphenyl)-1,6-naphthyridin-3,8-dicarbonsäurediethylester (1.x), Schmp. 140–141°C aus n-Hexan

(±)-1,4-Dihydro-5-isopropoxy-2-methyl-4-(2-trifluormethylphenyl)-1,6-naphthyridin-3-carbonsäureethylester · Hydrochlorid (1.y), Schmp. 137°C aus Ethylacetat

(±)-1,4-Dihydro-5-isopropoxy-4-(2-methoxyphenyl)-2-methyl-1,6-naphthyridin-3-carbonsäureethylester (1.z), Schmp. 145–146°C aus n-Hexan/Diisopropylether

(±)-1,4-Dihydro-5-isopropoxy-2-methyl-4-(2-thienyl)-1,6-naphthyridin-3-carbonsäureethylester (1.aa), Schmp. 110–111°C aus n-Hexan

(±)-4-(2-Cyanophenyl)-1,4-dihydro-5-isopropoxy-2-methyl-1,6-naphthyridin-3-carbonsäureethylester (1.ab), Schmp. 182–183°C aus n-Hexan/Diisopropylether

(±)-5-Benzyloxy-1,4-dihydro-2-methyl-4-(2-trifluormethylphenyl)-1,6-naphthyridin-3-carbonsäureethylester (1.ac), Schm. 142–143°C aus n-Hexan/Diisopropylether

(±)-2-Ethyl-1,4-dihydro-5-isopropoxy-8-methyl-4-(2-trifluormethylphenyl-1,6-naphthyridin-3-carbonsäureethylester (1.ad), Schm. 112–113°C aus n-Hexan

(±)-1,4-Dihydro-5-isopropoxy-2-methyl-4-(2-trifluormethylphenyl)-1,6-naphthyridin-3-carbonsäurebenzylester (1.ae), Schmp. 126–127°C aus n-Hexan

(±)-1,4-Dihydro-5-isopropoxy-2-methyl-4-(2-trifluormethylphenyl)-1,6-naphthyridin-3-carbonsäure-(2-dimethylaminoethyl)-ester (1.af), Schmp. 104–105°C aus n-Hexan

(±)-1,4-Dihydro-5-isopropoxy-2-methyl-4-(3-nitrophenyl)-1,6-naphthyridin-3-carbonsäure-(3-dimethylaminopropyl)ester (1.ag), Schmp. 134–136°C aus n-Hexan/Diisopropylether

(±)-4-(2-Difluoromethoxyphenyl)-1,4-dihydro-5-isopropoxy-2-methyl-1,6-naphthyridin-3-carbonsäureethylester (1.ah)

(±)-1,4-Dihydro-5-isopropoxy-2-methyl-4-(2-trifluormethylphenyl)-1,6-naphthyridin-3-carbonsäure-(2-dibenzylaminoethyl)ester (1.ai)

(±)-1,4-Dihydro-5-isopropoxy-2-methyl-4-(2-methylphenyl)-1,6-naphthyridin-3-carbonsäureethylester (1.aj), Schmp. 122–124°C aus n-Hexan/Diisopropylether

(±)-1,4-Dihydro-5-isopropoxy-2-methyl-4-(3-nitrophenyl)-1,6-naphthyridin-3-carbonsäure-(2-dimethylaminoethyl)ester (1.ak), Schmp. 91–93°C aus n-Hexan/Diisopropylether

Beispiel 2

(±)-1,4-Dihydro-5-methoxy-2-methyl-4-phenyl-1,6-naphthyridin-3-carbonsäuremethylester

5 g (17 mMol) (±)-1,4,5,6-Tetrahydro-2-methyl-5-oxo-4-phenyl-1,6-naphthyridin-3-carbonsäuremethylester und 5 g (34 mMol) Trimethyloxoniumtetrafluoroborat werden in 200 ml 1,2-Dichlorethan unter Stickstoffatmosphäre 1,5 Stunden bei Raumtemperatur gerührt. Es wird mit 50 ml Wasser ausgeschüttelt, die organische Phase abgetrennt und einrotiert. Durch Umkristallisation des Rückstands aus Isopropanol wird das Tetrafluoroborat der gewünschten Verbindung erhalten. Dieses wird mit gesättigter Kaliumhydrogencarbonatlösung und Ether verrührt, die Etherlösung abgetrennt, über Natriumsulfat getrocknet und einrotiert. Kristallisation der freien Base aus 50 ml n-Hexan/Diisopropylether 2:1 liefert farblose Kristalle vom Schmp. 210–212°C. Der als Ausgangsprodukt verwendete (±)-1,4,5,6-Tetrahydro-2-methyl-5-oxo-4-phenyl-1,6-naphthyridin-3-carbonsäuremethylester wird in Analogie zu Beispiel 1 unter Verwendung von 1,4-Dihydro-2,6-dimethyl-4-phenyl-pyridin-3,5-dicarbonsäuredimethylester hergestellt.

In analoger Weise erhält man die folgenden Verbindungen:

(±)-4-(2-Chlorphenyl)-1,4-dihydro-5-methoxy-2-methyl-1,6-naphthyridin-3-carbonsäureethylester (2.a), Schmp. 173–174°C aus n-Hexan/Diisopropylether

(±)-1,4-Dihydro-5-methoxy-2-methyl-4-(3-nitrophenyl)-1,6-naphthyridin-3-carbonsäureethylester (2.b), Schmp. 184–186°C aus Diisopropylether/Ethanol

(±)-4-(2-Fluorphenyl)-1,4-dihydro-5-methoxy-2-methyl-1,6-naphthyridin-3-carbonsäureethylester (2.c), Schmp. 148–150°C aus n-Hexan

(±)-1,4-Dihydro-5-methoxy-2-methyl-4-(2-trifluormethylphenyl)-1,6-naphthyridin-3-carbonsäureethylester (2.d), Schmp. 118–120°C aus n-Hexan

(±)-4-(3-Chlor-2-fluorphenyl)-1,4-dihydro-5-methoxy-2-methyl-1,6-naphthyridin-3-carbonsäuremethylester (2.e), Schmp. 214–216°C aus Diisopropylether/Methanol

(±)-4-(2-Bromphenyl)-1,4-dihydro-5-methoxy-2-methyl-1,6-naphthyridin-3-carbonsäuremethylester (2.f), Schmp. 204–205 °C aus Diisopropylether/Methanol

Beispiel 3
(±)-1,4-Dihydro-5-isopropoxy-2-methyl-4-(2-trifluormethylphenyl)-1,6-naphthyridin-3-carbonsäure

3,0 g (6,2 mMol) ±-1,4-Dihydro-5-isopropoxy-2-methyl-4-(2-trifluormethylphenyl)-1,6-naphthyridin-3-carbonsäurebenzylester werden mit 1.5 g 10% igem Palladium auf Aktivkohle in 100 ml Ethanol bei Normaldruck und Raumtemperatur hydriert. Nach 30 Minuten ist die Wasserstoffaufnahme beendet. Der Katalysator wird abfiltriert, das Lösungsmittel im Vakuum abdestilliert und der farblose, kristalline Rückstand aus Diisopropylether/Ethylacetat umkristallisiert. Man erhält (±)-1,4-Dihydro-5-isopropoxy-2-methyl-4-(2-trifluormethylphenyl)-1,6-naphthyridin-3-carbonsäure in Form farbloser Kristalle vom Schmp. 164–166 °C (Z). Der als Ausgangsprodukt verwendete (±)-1,4-Dihydro-5-isopropoxy-2-methyl-4-(2-trifluormethylphenyl)-1,6-naphthyridin-3-carbonsäurebenzylester wird in Analogie zu Beispiel 1 unter Verwendung von 1,4-Dihydro-2,6-dimethyl-4-(2-trifluormethylphenyl)-pyridin-3,5-dicarbonsäuredibenzylester hergestellt.

Beispiel 4
(±)-1,4-Dihydro-5-isopropoxy-2-methyl-4-(2-trifluormethylphenyl)-1,6-naphthyridin-3-carbonsäureethylester

12,5 g (33 mMol) (±)-1,4,5,6-Tetrahydro-2-methyl-5-oxo-4-(2-trifluormethylphenyl)-1,6-naphthyridin-3-carbonsäureethylester werden portionsweise bei Raumtemperatur zu einer Suspension von 1,3 g (43 mMol) Natriumhydrid (80%ig in Öl) in 200 ml trockenem Dimethylformamid gegeben. Nach beendeter Wasserstoffentwicklung wird 15 Minuten bei Raumtemperatur gerührt, dann 7,2 g (43 mMol) Isopropyljodid zugegeben und 3 Tage bei dieser Temperatur weitergerührt. Es wird im Vakuum eingedampft, der Rückstand mit 500 ml Wasser versetzt und 30 Minuten im Ultraschallbad behandelt. Die ausgefallenen Kristalle werden abfiltriert und bei 50 °C getrocknet.

Dieses rohe Produktgemisch wird in wenig Ethylacetat gelöst und an Kieselgel mit Toluol/Ethylacetat 3:1 chromatographiert. Die Fraktionen mit dem $R_f$ 0,4 werden im Vakuum eingedampft und der Rückstand mit n-Hexan bis zur Kristallisation verrührt. Das gebildete Produkt wird abfiltriert und aus 60 ml n-Hexan umkristallisiert.

Man erhält farblose Kristalle vom Schmp. 102–103 °C.

Der als Ausgangsprodukt verwendete (±)-1,4,5,6-Tetrahydro-2-methyl-5-oxo-4-(2-trifluormethylphenyl)-1,6-naphthyridin-3-carbonsäureethylester wird wie folgt hergestellt:

6,0 g (0,2 Mol) Natriumhydrid (80%ig in Öl) werden in 100 ml trockenem Dimethylformamid suspendiert und unter Stickstoffatmosphäre eine Lösung von 79,2 g (0,2 Mol) 1,4-Dihydro-2,6-dimethyl-4-(2-trifluormethylphenyl)pyridin-3,5-dicarbonsäurediethylester in 400 ml Dimethylformamid bei Raumtemperatur zugetropft. Nach Beendigung der Wasserstoffentwicklung wird noch 10 Minuten bei Raumtemperatur gerührt, dann eine Lösung von 16,2 g (0,2 Mol) s-Triazin in 300 ml Dimethylformamid zugetropft und anschliessend 16 Stunden bei 110 °C gerührt. Nach dem Abkühlen wird die Reaktionslösung im Vakuum eingedampft, der Rückstand mit 1,5 l Aceton verrührt, vom Ungelösten filtriert, die Acetonlösung im Vakuum eingedampft und der Rückstand an Kieselgel mit Dichlormethan-Methanol 9:1 chromatographiert. Die Fraktion mit dem $R_f$ 0,45 wird isoliert, mit 200 ml Chloroform verrührt und die nicht gelösten, hellbeigen Kristalle abfiltriert.

Zur weiteren Reinigung wird aus Ethanol umkristallisiert. Man erhält farblose Kristalle vom Schmp. 261 C.

Der als Ausgangsprodukt verwendete 1,4-Dihydro-2,6-dimethyl-4-(2-trifluormethylphenyl)pyridin-3,5-dicarbonsärediethylester wird wie folgt hergestellt:

50 g (0,29 Mol) 2-Trifluormethylbenzaldehyd, 76 g (0,58 Mol) Acetessigsäureethylester und 30 ml konz. wässrige Ammoniaklösung werden in 200 ml Ethanol gelöst und 16 Stunden zum Sieden erhitzt. Das nach dem Abkühlen ausgefallene Produkt wird abfiltriert und mit kaltem Ethanol nachgewaschen.

Man erhält blassgelbe Kristalle vom Schmp. 142–143 °C.

Die folgenden Vergleichsversuche veranschaulichen die pharmakologische Wirksamkeit der Verbindungen gemäss der allgemeinen Formel I:

a) Isolierte glatte Muskeln

von Kaninchen (Gefässringe, A. basilaris, A. coronaria, A. saphena) werden im Organbad so eingespannt, dass isometrische Kontraktionen gemessen werden können. Die Kontraktion wird durch eine Kaliumdepolarisation in Tyrodelösung ausgelöst. Diese Versuchsanordnung ist ein bekanntes Standardmodell zur Erkennung von Substanzen, die die in der Kaliumdepolarisation geöffneten Calciumkanäle blockieren (Fleckenstein, 1983). Wie aus Tabelle I hervorgeht, wirken einige Substanzen im nanomolaren Bereich halbmaximal erschlaffend. Diese Potenz übertrifft teilweise die der schon bekannten Calciumantagonisten Diltiazem und Nifedipin erheblich.

b) Isolierte Papillarmuskel

Papillarmuskel aus der linken Herzkammer des Meerschweinchens werden wie bei isolierten Gefässen im Organbad zur isometrischen Kontraktionsmessung eingespannt und durch Feldreizung mit einer Frequenz von 250/min (Reizdauer 10 msec, Amplitude supramaximal) elektrisch gereizt. Die Tabelle II zeigt, dass die Substanzen, die ihre Ca-antagonistische Wirkung selektiv am glatten Gefässmuskel entfalten und am Myokard überraschend im therapeutischen Bereich (1–100 nmol) keine negativ, teilweise sogar positiv inotrope Wirkung zeigen. Es ist allerdings nicht auszuschliessen, dass das als Lösungsmittel verwendete Dimethlysulfoxid zur beobachteten positiven Inotropie beigetragen hat. Die Verbindung des Beispiels 1c besitzt bei einer therapeutischen Konzentration von 3 x $10^{-7}$ Mol/l eine maximale Zunahme der Kontraktilität von 36%, bei einer Konzentration von 4 x $10^{-4}$ Mol/l, die für die Therapie irrelevant sind, zeigen die Verbindungen 1, 1a und 1h jedoch, wie erwartet, schwach negativ inotrope Wirkungen. Im therapeutischen Bereich dürften negativ inotrope Wirkungen jedoch nicht auftreten.

Tabelle I zeigt Konzentrationen ($IC_{50}$, mol/l) von Verbindungen I, die eine halbmaximale Hemmung der $K^+$-Depolarisationskontraktur der Gefässringe im Organbad bewirken. A. saph. = Arteria saphena des Kaninchens; mittlerer Durchmesser 0,5-1,0 mm. Zum Vergleich werden die $IC_{50}$-Werte der Calciumantagonisten Diltiazem und Nifedipin angegeben.

Tabelle I

| Beispiel | A. bas. | A. cor. | A. saph. |
|---|---|---|---|
| 1 | $5\times10^{-9}$ | $3\times10^{-8}$ | $7\times10^{-9}$ |
| 1a | $2,4\times10^{-9}$ | $2,7\times10^{-9}$ | $8\times10^{-9}$ |
| 1h | $3\times10^{-6}$ | $1,5\times10^{-8}$ | $1\times10^{-6}$ |
| 1b | $6,6\times10^{-10}$ | $7,5\times10^{-10}$ | $3,4\times10^{-9}$ |
| 1c | $1,5\times10^{-8}$ | $6,5\times10^{-8}$ | $1\times10^{-6}$ |
| 1g | $5,9\times10^{-9}$ | $3,2\times10^{-9}$ | - |
| 1l | $7,4\times10^{-9}$ | $3,1\times10^{-8}$ | $3,9\times10^{-7}$ |
| 1p | $1,6\times10^{-8}$ | $5,3\times10^{-8}$ | - |
| 1q | $3,5\times10^{-8}$ | $1,9\times10^{-7}$ | - |
| 2 | $2,5\times10^{-7}$ | $1\times10^{-6}$ | $9\times10^{-8}$ |
| 2a | $6\times10^{-9}$ | $1\times10^{-8}$ | $1\times10^{-7}$ |
| 2b | $1,8\times10^{-9}$ | $1,2\times10^{-8}$ | $6\times10^{-7}$ |
| Diltiazem | $1,2\times10^{-7}$ | $1,7\times10^{-7}$ | $2,9\times10^{-6}$ |
| Nifedipin | $2,7\times10^{-9}$ | $5\times10^{-9}$ | $5,9\times10^{-8}$ |

Änderungen der Kontraktionsamplitude von isolierten Papillarmuskeln des Meerschweinchens (Reizfrequenz 250/min. Reizdauer 10 msec., Reizamplitude 10-20 V Feldreizung).

IC = Hemmkonzentration (inhibitory concentration), $IC_{100}$ entspricht maximaler Wirkung. Δ% = maximale Abnahme der Kontraktilität.

Die Hemmkonzentrationen von Diltiazem und Nifedipin sind zum Vergleich mit aufgeführt.

Tabelle II

| Beispiel | Zahl der Tiere | $IC_{100}$ | Δ% |
|---|---|---|---|
| 1 | (n = 7) | $3\times10^{-4}$ | -40 |
| 1a | (n = 4) | $3\times10^{-4}$ | -48 |
| 1g | | $1\times10^{-4}$ | -38 |
| 1l | | $1,2\times10^{-4}$ | -38 |
| 1p | | $1\times10^{-5}$ | -23 |
| 1q | | $1\times10^{-5}$ | -7 |
| 1h | (n = 4) | $3\times10^{-4}$ | -54 |

Tabelle 2 (Fortsetzung)

| | | | |
|---|---|---|---|
| Diltiazem | (n = 6) | $10^{-5}$ | -60 |
| Nifedipin* | | $10^{-6}$ | -80 |

* Hof und Scholtysik, J. Cardiovasc. Pharmacol. 5: 176-183 (1983), Versuche am Papillarmuskel des Kaninchens

Die Tabelle II zeigt die maximalen Wirkungen auf die Kontraktilität bei der jeweils hierfür nötigen Konzentration (IC 100 = Inhibitory Concentration). Hieraus ist ersichtlich, dass die Vergleichsverbindungen bereits bei erheblich niedrigeren Konzentrationen (IC 100) eine höhere Abnahme der Kontraktilität zeigen. Die Konzentration IC 100 liegt weit ausserhalb des therapeutischen Bereichs der erfindungsgemässen Verbindung, so dass dort eine negative Inotropie nicht zu finden ist. Daraus folgt, dass die therapeutische Sicherheit der erfindungsgemässen Verbindung gegenüber dem Stand der Technik erheblich gesteigert werden konnte.

**Patentansprüche für die Vertragsstaaten: BE, CH, DE, FR, GB, IT, LI, LU, NE, SE**

1. 1,6-Naphthyridin-Derivate der allgemeinen Formel I

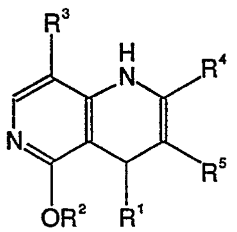

(I)

in welcher $R^1$ einen unsubstituierten oder durch Halogenatome, Niederalkyl, Niederalkoxy-, Niederalkylendioxy-, Nitro-, Cyano-, Niederalkylamino-, Methylthio-, Trifluormethyl- oder Difluormethoxygruppen mono- oder disubstituierten Phenylring oder einen Thienyl-, Pyridyl- oder 2,1,3-Benzoxadiazolylring; $R^2$ eine geradkettige oder verzweigte Alkylgruppe mit bis zu vier Kohlenstoffatomen oder eine Benzylgruppe; $R^3$ ein Wasserstoffatom oder eine geradkettige oder verzweigte Alkyl- oder Alkoxycarbonylgruppe mit jeweils bis zu vier Kohlenstoffatomen in der Alkylgruppe; $R^4$ eine geradkettige oder verzweigte Alkylgruppe mit bis zu vier Kohlenstoffatomen und $R^5$ einen Carboxyl- oder Alkoxycarbonylrest der allgemeinen Formel III

$$-CO_2R^7 \qquad (III)$$

in welcher $R^7$ entweder Wasserstoff, einen Methyl-, Ethyl-, Propyl-, Isopropyl-, n-Butyl-, Isobutyl-, sec. Butyl-, tert. Butyl-, Benzyl-, 2-Piperidinoethylrest, eine Alkoxyalkyl- oder Alkylthioalkylgruppe der allgemeinen Formeln IV und V

$$-(CH_2)_n-O-R^8 \qquad (IV)$$

$$-(CH_2)_n-S-R^8 \qquad (V)$$

worin $R^8$ eine niedere Alkylgruppe mit 1 bis 3 Kohlenstoffatomen und n die Zahlen 2 oder 3 bedeutet, oder eine Aminoalkylgruppe der allgemeinen Formel VI

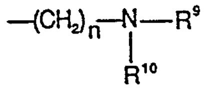

(VI)

worin $R^9$ und $R^{10}$ gleich oder verschieden sein können und Wasserstoff, eine geradkettige oder verzweigte niedere Alkylgruppe mit 1 bis 4 Kohlenstoffatomen oder eine Benzylgruppe darstellen und n gleich 2 oder 3 ist, bedeuten, sowie gegebenenfalls deren pharmakologisch unbedenkliche Salze.

2. 1,6-Naphthyridin-Derivate der allgemeinen Formel I gemäss Anspruch 1, dadurch gekennzeichnet, dass $R^1$ einen unsubstituierten oder durch Halogen, Methyl, Methoxy, Nitro, Cyano, Trifluormethyl oder Difluormethoxy substituierten Phenylrest oder einen Thienyl-, Pyridyl- oder 2,1,3-Benzoxadiazolylrest; $R^2$ einen Methyl-, Ethyl-, n-Propyl-, Isopropyl, n-Butyl-, sek. Butyl-, Isobutyl- oder Benzylrest; $R^3$ Wasserstoff, eine Methyl-, Ethyl- oder Isopropylgruppe oder einen Methoxycarbonyl-, Ethoxycarbonyl-, Propoxycarbonyl- oder Isopropoxycarbonylrest; $R^4$ einen Methyl- oder Ethylrest und $R^5$ einen Carboxyl- oder Alkoxycarbonylrest der allgemeinen Formel III, in welcher $R^7$ entweder Wasserstoff, einen Methyl-, Ethyl-, Propyl-, Isopropyl-, n-Butyl-, Isobutyl-, sec. Butyl-, tert.Butyl-, Benzyl-, 2-Methylthioethyl-, 2-Methoxyethyl-, 2-Piperidinoethylrest oder eine Aminoalkylgruppe der allgemeinen Formel VI, worin $R^9$ und $R^{10}$ gleich oder verschieden sein können und Wasserstoff, eine geradkettige oder verzweigte Alkylgruppe mit 1 bis 4 C-Atomen oder eine Benzylgruppe darstellen und n die Zahlen 2 oder 3 bedeuten.

3. 1,6-Naphthyridin-Derivate gemäss Anspruch 1, dadurch gekennzeichnet, dass $R^1$ einen Phenyl-, 2-Chlorphenyl-, 2-Bromphenyl-, 2-Fluorphenyl, 2-Nitrophenyl-, 2-Trifluormethylphenyl, 2-Cyanophenyl-, 2-Methylphenyl-, 2-Difluormethoxyphenyl-, 2-Methoxyphenyl-, 3-Nitrophenyl-, 2,3-Dichlorphenyl-, 2-Chlor-6-Fluorphenyl-, 3-Chlor-2-Fluorphenyl-, 2-Thienyl-, Pyridyl- oder 2,1,3-Benzoxadiazolylrest; $R^2$ einen Methyl-, Ethyl-, n-Propyl-, Isopropyl, n-Butyl-, sek. Butyl-, Isobutyl- oder Benzylrest; $R^3$ Wasserstoff, eine Methyl- oder Ethoxycarbonylrest; $R^4$ einen Methyl- oder Ethylrest und $R^5$ einen Methoxycarbonyl-, Ethoxycarbonyl-, Propoxycarbonyl-, Isopropoxycarbonyl-, Butoxycarbonyl-, Isobutoxycarbonyl-, sec. Butoxycarbonyl-, tert. Butoxycarbonyl-, Benzyloxycarbonyl-, (2-Methoxyethyl)oxycarbonyl-, (2-Methylthioethyl)oxycarbonyl-, (2-Piperidinoethyl)oxycarbonyl-, (2-(N-Methyl-N-phenylamino-ethyl)oxycarbonyl-, (2-Diethylaminoethyl)oxycarbonyl-, (2-Aminoethyl)oxycarbonyl-, (2-Dimethylaminoethyl)oxycarbonyl-, (3-Dimethylaminopropyl)oxycarbonyl-, (2-Dibenzylaminoethyl)oxycarbonyl-, (2-(N-Benzyl-N-methylamino)ethyl)oxycarbonyl oder (3-(N-Benzyl-N-methylamino)ethyl)oxycarbonyl oder (3-(N-Benzyl-N-methylamino)propyl)oxycarbonylrest bedeuten.

4. (±)-1,4-Dihydro-5-isopropoxy-2-methyl-4-(2-trifluormethylphenyl)-1,6-naphthyridin-3-carbonsäureethylester und dessen pharmakologisch verträgliche Salze.

5. (±)-1,4-Dihydro-5-isopropoxy-2-methyl-4-(-2-trifluormethylphenyl)-1,6-naphthyridin-3-carbonsäure-[2-(N-benzyl-N-methylamino)ethyl]-ester und dessen pharmakologisch verträgliche Salze.

6. Verbindungen gemäss Anspruch 1 bis 5 in Form der Enantiomeren.

7. Verfahren zur Herstellung von 1,6-Naphthyridin-Derivaten der allgemeinen Formel I gemäss Anspruch 1 bis 6, dadurch gekennzeichnet, dass man 1,6-Naphthyridinon-Derivate der allgemeinen Formel VII

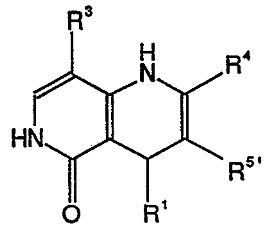

(VII)

in welcher $R^1$, $R^3$ und $R^4$ die oben angegebene Bedeutung haben und $R^5$ die oben genannte Bedeutung mit Ausnahme einer Carboxylgruppe hat, in an sich bekannter Weise alkyliert zu Verbindungen der allgemeinen Formel I in welcher $R^1$, $R^3$ und $R^4$ die oben angegebene Bedeutung haben und $R^5$ die oben genannte Bedeutung mit Ausnahme einer Carboxylgruppe hat und gegebenenfalls in deren pharmakologisch verträgliche Salze überführt.

8. Verfahren zur Herstellung von 1,6-Naphthyridin-Derivaten der allgemeinen Formel I gemäss Anspruch 1 bis 5, in welcher $R^1$, $R^3$ und $R^4$ die oben angegebene Bedeutung haben und $R^5$ eine Carboxylgruppe ist, dadurch gekennzeichnet, dass man 1,6-Naphthyridinon-Derivate der allgemeinen Formel I, in welcher $R^1$, $R^3$ und $R^4$ die oben angegebene Bedeutung haben und $R^5$ einen Benzyloxycarbonylrest bedeutet, in an sich bekannter Weise hydrogenolytisch spaltet und gegebenenfalls in deren pharmakologisch verträgliche Salze überführt.

9. Arzneimittel mit einem Gehalt an mindestens einem 1,6-Naphthyridin-Derivat gemäss Anspruch 1 bis 6 als Wirkstoff.

10. Verwendung von 1,6-Naphthyridin-Derivaten gemäss Anspruch 1 bis 6 zur Herstellung von Arzneimitteln zur Bekämpfung von cardialen, cerebralen und peripheren Gefässerkrankungen, Ischämien, Infarkten, Thrombosen, Stenosen, Arteriosklerosen und zur Bekämpfung von Herz-Kreislauf-Erkrankungen.

**Patentansprüche für den Vertragsstaat: AT**

1. Verfahren zur Herstellung von 1,6-Naphthyridin-Derivaten der allgemeinen Formel I

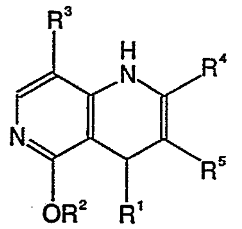

(I)

in welcher $R^1$ einen unsubstituierten oder durch Halogenatome, Niederalkyl, Niederalkoxy-, Niederalkylendioxy-, Nitro-, Cyano-, Niederalkylamino-, Methylthio-, Trifluormethyl- oder Difluormethoxygruppen mono- oder disubstituierten Phenylring oder einen Thienyl-, Pyridyl- oder 2,1,3-Benzoxadiazolylring; $R^2$ eine geradkettige oder verzweigte Alkylgruppe mit bis zu vier Kohlenstoffatomen oder eine Benzylgruppe; $R^3$ ein Wasserstoffatom oder eine geradkettige oder verzweigte Alkyl- oder Alkoxycarbonylgruppe mit jeweils bis zu vier Kohlenstoffatomen in der Alkylgruppe; $R^4$ eine geradkettige oder verzweigte Alkylgruppe mit bis zu vier Kohlenstoffatomen und $R^5$ einen Carboxyl- oder Alkoxycarbonylrest der allgemeinen Formel III

$$-CO_2R^7 \qquad (III)$$

in welcher $R^7$ entweder Wasserstoff, einen Methyl-, Ethyl-, Propyl-, Isopropyl-, n-Butyl-, sec. Butyl-, tert. Butyl-, Benzyl-, 2-Piperidinoethylrest, eine Alkoxyalkyl- oder Alkythioalkylgruppe der allgemeinen Formeln IV und V

$$-(CH_2)_n-O-R^8 \qquad (IV)$$
$$-(CH_2)_n-S-R^8 \qquad (V)$$

worin $R^8$ eine niedere Alkylgruppe mit 1 bis 3 Kohlenstoffatomen und n die Zahlen 2 oder 3 bedeutet, oder eine Aminoalkylgruppe der allgemeinen Formel VI

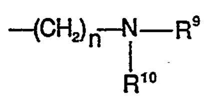

(VI)

worin $R^9$ und $R^{10}$ gleich oder verschieden sein können und Wasserstoff, eine geradkettige oder verzweigte niedere Alkylgruppe mit 1 bis 4 Kohlenstoffatomen oder eine Benzylgruppe darstellen und n gleich 2 oder 3 ist, bedeuten, als Racemat oder in Form der Enantiomeren, sowie gegebenenfalls deren pharmakologisch unbedenkliche Salze, dadurch gekennzeichnet, dass man 1,6-Naphthyridinon-Derivate der allgemeinen Formel VII

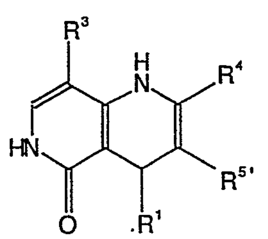

(VII)

in welcher $R^1$, $R^3$ und $R^4$ die oben angegebene Bedeutung haben und $R^5$ die oben genannte Bedeutung mit Ausnahme einer Carboxylgruppe hat, in an sich bekannter Weise alkyliert zu Verbindungen der allgemeinen Formel I in welcher $R^1$, $R^3$ und $R^4$ die oben angegebene Bedeutung haben und $R^5$ die oben genannte Bedeutung mit Ausnahme einer Carboxylgruppe hat und gegebenenfalls in deren pharmakologisch verträgliche Salze überführt.

2. Verfahren zur Herstellung von 1,6-Naphthyridin-Derivaten der allgemeinen Formel I gemäss Anspruch 1, in welcher $R^1$, $R^3$ und $R^4$ die oben angegebene Bedeutung haben und $R^5$ eine Carboxylgruppe ist, dadurch gekennzeichnet, dass man 1,6-Naphthyridinon-Derivate der allgemeinen Formel I, in welcher $R^1$, $R^3$ und $R^4$ die oben angegebene Bedeutung haben und $R^5$ einen Benzyloxycarbonylrest bedeutet, in an sich bekannter Weise hydrogenolytisch spaltet und gegebenenfalls in deren pharmakologisch verträgliche Salze überführt.

3. Verfahren gemäss Anspruch 1 oder 2 zur Herstellung von 1,6-Naphthyridin-Derivaten der allgemeinen Formel I gemäss Anspruch 1, dadurch gekennzeichnet, dass $R^1$ einen unsubstituierten oder durch Halogen, Methyl, Methoxy, Nitro, Cyano, Trifluormethyl oder Difluormethoxy substituierten Phenylrest oder einen Thienyl-, Pyridiyl- oder 2,1,3-Benzoxadiazolylrest; $R^2$ einen Methyl-, Ethyl-, n-Propyl-, Isopropyl, n-Butyl-, sek. Butyl-, Isobutyl- oder Benzylrest; $R^3$ Wasserstoff, eine Methyl-, Ethyl- oder Isopropylgruppe oder einen Methoxycarbonyl-, Ethoxycarbonyl-, Propoxycarbonyl- oder Isopropoxycarbonylrest; $R^4$ einen Methyl- oder Ethylrest und $R^5$ einen Carboxyl- oder Alkoxycarbonylrest der allgemeinen Formel III, in welcher $R^7$ entweder Wasserstoff, einen Methyl-, Ethyl-, Propyl-, Isopropyl-, n-Butyl-, Isobutyl-, sec. Butyl-, tert. Butyl-, Benzyl-, 2-Methylthioethyl-, 2-Methoxyethyl- 2-Piperidinoethylrest oder eine Aminoalkylgruppe der allgemeinen Formel VI, worin $R^9$ und $R^{10}$ gleich oder verschieden sein können und Wasserstoff, eine geradkettige oder verzweigte Alkylgruppe mit 1 bis 4 C-Atomen oder eine Benzylgruppe darstellen und n die Zahlen 2 oder 3 bedeuten.

4. Verfahren gemäss Anspruch 1 oder 2 zur Herstellung von 1,6-Naphthyridin-Derivaten gemäss Anspruch 1, dadurch gekennzeichnet, dass $R^1$ einen Phenyl-, 2-Chlorphenyl-, 2-Bromphenyl-, 2-Fluorphenyl, 2-Nitrophenyl-, 2-Trifluormethylphenyl, 2-Cyanophenyl-, 2-Methylphenyl-, 2-Difluormethoxyphenyl-, 2-Methoxyphenyl-, 3-Nitrophenyl-, 2,3-Dichlorphenyl-, 2-Chlor-6-Fluorphenyl-, 3-Chlor-2-Fluorphenyl-, 2-Thienyl-, Pyridyl- oder 2,1,3-Benzoxadiazolylrest; $R^2$ einen Methyl-, Ethyl-, n-Propyl-, Isopropyl, n-Butyl-, sek. Butyl-, Isobutyl- oder Benzylrest; $R^3$ Wasserstoff, eine Methyl- oder Ethoxycarbonylrest; $R^4$ einen Methyl- oder Ethylrest und $R^5$ einen Methoxycarbonyl-, Ethoxycarbonyl-, Propoxycarbonyl-, Isopropoxycarbonyl-, Butoxycarbonyl-, Isobutoxycarbonyl-, sec. Butoxycarbonyl-, tert. Butoxycarbonyl-, Benzyloxycarbonyl-, (2-Methoxyethyl)oxycarbonyl-, (2-Methylthioethyl)oxycarbonyl-, (2-Piperidinoethyl)oxycarbonyl-, (2-N-Methyl-N-phenylamino)ethyl)oxycarbonyl-, (2-Diethylaminoethyl)oxycarbonyl-, (2-Aminoethyl)oxycarbonyl-, (2-Dimethylaminoethyl)oxycarbonyl-, (3-Dimethylaminopropyl)oxycarbonyl-, (2-Dibenzylaminoethyl)oxycarbonyl-, (2-N-Benzyl-N-methylamino)ethyl)oxycarbonyl oder (3-N-Benzyl-N-methylamino)propyl)oxycarbonylrest bedeuten.

5. Verfahren gemäss Anspruch 1 oder 2 zur Herstellung von (±)-1,4-Dihydro-5-isopropoxy-2-methyl-4-(2-trifluormethylphenyl)-1,6-naphthyridin-3-carbonsäureethylester und dessen pharmakologisch verträgliche Salze und dessen Enantiomere.

6. Verfahren gemäss Anspruch 1 oder 2 zur Herstellung von (±)-1,4-Dihydro-5-isopropoxy-2-methyl-4-(2-trifluormethylphenyl)-1,6-naphthyridin-3-carbonsäure-[2-(N-benzyl-N-methylamino) ethyl]ester, dessen pharmakologisch verträgliche Salze und dessen Enantiomere.

7. Verwendung von 1,6-Naphthyridin-Derivaten gemäss Anspruch 1 bis 6 zur Herstellung von Arzneimitteln zur Bekämpfung von cardialen, cerebralen und peripheren Gefässerkrankungen, Ischämien, Infarkten, Thrombosen, Stenosen, Arteriosklerosen und zur Bekämpfung von Herz-Kreislauf-Erkrankungen.

**Claims for the Contracting States: BE, CH, DE, FR, GB, IT, LI, LU, NE, SE**

1. 1,6-Naphthyridine derivatives of the general formula:

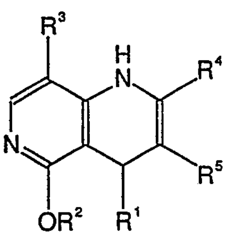

(I)

wherein $R^1$ is an unsubstituted phenyl radical or a mono- or disubstituted phenyl radical substituted by halogen atoms, lower alkyl, lower alkoxy, lower alkylenedioxy, nitro, cyano, lower alkylamino, methylthio, trifluormethyl or difluoromethoxy radicals or is a thienyl, pyridyl or 2,1,3-benzoxadiazolyl radical; $R^2$ is a straight-chained or branched alkyl radical containing up to 4 carbon atoms or a benzyl radical; $R^3$ is a hydrogen atom or a straight-chained or branched alkyl or alkoxycarbonyl radical containing up to 4 carbon atoms in each case in the alkyl radical; $R^4$ is a straight-chained or branched alkyl radical containing up to 4 carbon atoms and $R^5$ is a carboxyl or alkoxycarbonyl radical of the general formula:

$$-CO_2R^7 \quad \text{(III)}$$

wherein $R^7$ is either hydrogen, a methyl, ethyl, propyl, isopropyl, n-butyl, isobutyl, sec.-butyl, tert.-butyl, benzyl, 2-piperidinoethyl radical, an alkoxyalkyl or alkylthioalkyl radical of the general formulae:

$$-CH_2)_n-O-R^8 \quad \text{(IV)}$$
$$-(CH_2)_n-S-R^8 \quad \text{(V)}$$

wherein $R^8$ is a lower alkyl radical with 1 to 3 carbon atoms and n is the number 2 or 3, or an aminoalkyl radical of the general formula:

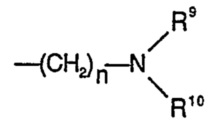

(VI)

wherein $R^9$ and $R^{10}$ may be the same or different and are hydrogen, a straight-chained or branched lower alkyl radical with 1 to 4 carbon atoms or a benzyl radical and n equals 2 or 3, as well as the pharmacologically acceptable salts thereof, if the case arises.

2. 1,6-Naphthyridine derivatives of the general formula I according to claim 1, characterised in that $R^1$ is an unsubstituted phenyl radical or a phenyl radical substituted by halogen, methyl, methoxy, nitro, cyano, trifluormethyl or difluoromethoxy or a thienyl, pyridyl or 2,1,3-benzoxadiazolyl radical; $R^2$ is a methyl, ethyl, n-propyl, isopropyl, n-butyl, sec.-butyl, isobutyl or benzyl radical; $R^3$ is hydrogen, a methyl, ethyl or isopropyl radical or a methoxycarbonyl, ethoxycarbonyl, propoxycarbonyl or isopropoxycarbonyl radical; $R^4$ is as methyl or ethyl radical and $R^5$ is a carboxyl or alkoxycarbonyl radical of the general formula III, wherein $R^7$ is either hydrogen, a methyl, ethyl, propyl, isopropyl, n-butyl, isobutyl, sec.-butyl, tert.-butyl, benzyl, 2-methylthioethyl, 2-methoxyethyl, 2-piperidinoethyl radical or an aminoalkyl radical of the general formula VI, wherein $R^9$ and $R^{10}$ may be the same or different and are hydrogen, a straight-chained or branched alkyl radical with 1 to 4 carbon atoms or a benzyl radical, and n is the number 2 or 3.

3. 1,6 Naphthyridine derivatives according to claim 1, characterised in that $R^1$ is a phenyl, 2-chlorphenyl, 2-bromophenyl, 2-fluorophenyl, 2-nitrophenyl, 2-trifluoromethylphenyl, 2-cyanophenyl, 2-methylphenyl, 2-difluoromethoxyphenyl, 2-methoxyphenyl, 3-nitrophenyl, 2,3-dichlorophenyl, 2-chloro-6-fluorophenyl, 3-chloro-2-fluorophenyl, 2-thienyl, pyridyl or 2,1,3-benzoxadiazolyl radical; $R^2$ is a methyl, ethyl, n-propyl, isopropyl, n-butyl, sec-butyl, isobutyl or benzyl radical; $R^3$ is hydrogen, a methyl or ethoxycarbonyl radical; $R^4$ is a methyl or ethyl radical and $R^5$ is a methoxycarbonyl, ethoxycarbonyl, propoxycarbonyl, isopropoxycarbonyl, butoxycarbonyl, isobutoxycarbonyl, sec.-butoxycarbonyl, tert.-butoxycarbonyl, benzyloxycarbonyl, (2-methoxyethyl)oxycarbonyl, (2-methylthioethyl)oxycarbonyl, (2-piperidinoethyl)oxycarbonyl, (2-(N-methyl-N-phenylamino)ethyl)-oxycarbonyl, (2-diethylaminoethyl)oxycarbonyl, (2-aminoethyl)oxycarbonyl, (2-dimethylaminoethyl)oxycarbonyl, (3-dimethylaminopropyl)oxycarbonyl, (2-dibenzylaminoethyl)oxycarbonyl,

(2-N-benzyl-N-methylamino)ethyl)oxycarbonyl or (3-N-benzyl-N-methylamino)propyl)oxycarbonyl radical.

4. Methyl (±)-1,4-dihydro-5-isopropoxy-2-methyl-4-(2-trifluoromethylphenyl)-1,6-naphthyridine-3-carboxylate and pharmacologically acceptable salts thereof.

5. 2-(N-benzyl-N-methylamino)-ethyl (±)-1,4-dihydro-5-isopropoxy-2-methyl-4-(2-trifluoromethylphenyl)-1,6-naphthyridine-3-carboxylate and pharmacologically acceptable salts thereof.

6. Compounds according to claims 1 to 5 in the form of the enantiomers.

7. Process for the preparation of 1,6-naphthyridine derivatives of the general formula I according to claims 1 to 6, characterised in that 1,6-naphthyridinone derivatives of the general formula:

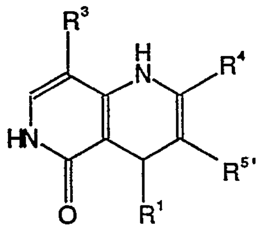

(VII)

wherein $R^1$, $R^3$ and $R^4$ have the above stated meaning and $R^5$ has the above mentioned meaning with the exception of a carboxyl radical, are alkylated in known manner into compounds of the general formula I in which $R^1$, $R^3$ and $R^4$ have the above stated meaning and $R^5$ has the above mentioned meaning with the exception of a carboxyl radical and are transformed into pharmacologically acceptable salts thereof, if the case arises.

8. Process for the preparation of 1,6-naphthyridine derivatives of the general formula I according to claims 1 to 5, in which $R^1$, $R^3$ and $R^4$ have the above stated meaning and $R^5$ is a carboxyl radical, characterised in that 1,6-naphthyridinone derivatives of the general formula I, in which $R^1$, $R^3$ and $R^4$ have the above stated meaning and $R^5$ is a benzyloxycarbonyl radical, are split hydrogenolytically in known manner and transformed into pharmacologically acceptable salts thereof, if the case arises.

9. Medicament with a content of at least one 1,6-naphthyridine derivative according to claims 1 to 6 as active ingredient.

10. Use of 1,6-naphthyridine derivatives according to claims 1 to 6 for the preparation of medicaments for combatting cardiac, cerebral and peripheral vascular diseases, ischaemiae, infarctions, thromboses, stenoses, arterial scleroses and for combatting cardiovascular diseases.

**Claims for the Contracting State: AT**

1. Process for the preparation of 1,6-naphthyridine derivatives of the general formula: wherein

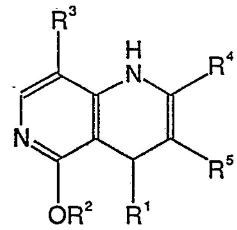

(I)

$R^1$ is an unsubstituted phenyl radical or a mono- or disubstituted phenyl radical substituted by halogen atoms, lower alkyl, lower alkoxy, lower alkylenedioxy, nitro, cyano, lower alkylamino, methylthio, trifluormethyl or difluoromethoxy radicals or is a thienyl, pyridyl or 2,1,3-benzoxadiazolyl radical; $R^2$ is a straight-chained or branched alkyl radical containing up to 4 carbon atoms or a benzyl radical; $R^3$ is a hydrogen atom or a straight-chained or branched alkyl or alkoxycarbonyl radical containing up to 4 carbon atoms in each case in the alkyl radical; $R^4$ is a straight-chained or branched alkyl radical containing up to 4 carbon atoms and $R^5$ is a carboxyl or alkoxycarbonyl radical of the general formula:

$$-CO_2R^7 \qquad (III)$$

wherein $R^7$ is either hydrogen, a methyl, ethyl, propyl, isopropyl, n-butyl, isobutyl, sec.-butyl, tert.-butyl, benzyl, 2-piperidinoethyl radical, an alkoxyalkyl or alkylthioalkyl radical of the general formulae:

$$-(CH_2)_n-O-R^8 \qquad (IV)$$
$$-(CH^2)^n-S-R^8 \qquad (V)$$

wherein $R^8$ is a lower alkyl radical with 1 to 3 carbon atoms and n is the number 2 or 3, or an amino alkyl radical of the general formula:

$$-(CH_2)_n-\underset{\substack{| \\ R^{10}}}{N}-R^9 \qquad (VI)$$

wherein $R^9$ and $R^{10}$ may be the same or different and are hydrogen, a straight-chained or branched lower alkyl radical with 1 to 4 carbon atoms or a benzyl radical and n equals 2 or 3, as racemate or in the form of the enantiomers, as well as pharmacologically acceptable salts thereof, if the case arises, characterised in that 1,6-naphthyridinone derivatives of the general formula:

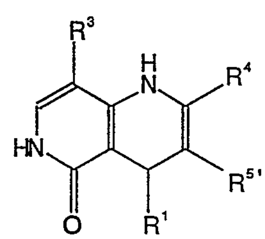

(VII)

wherein $R^1$, $R^3$ and $R^4$ have the above stated meaning and $R^5$ has the above mentioned meaning with the exception of a carboxyl radical, are alkylated in known manner into compounds of the general formula I in which $R^1$, $R^3$ and $R^4$ have the above stated meaning and $R^5$ has the above mentioned meaning with the exception of a carboxyl radical, and are transformed into pharmacologically acceptable salts thereof, if the case arises.

2. Process for the preparation of 1,6-naphthyridine derivatives of the general formula I according to claim 1, in which $R^1$, $R^3$ and $R^4$ have the above stated meaning and $R^5$ is a carboxyl radical, characterised in that 1,6-naphthyridinone derivatives of the general formula I, in which $R^1$, $R^3$ and $R^4$ have the above stated meaning and $R^5$ is a benzyloxycarbonyl radical, are split hydrogenolytically in known manner and transformed into pharmacologically acceptable salts thereof, if the case arises.

3. Process according to claim 1 or 2 for the preparation of 1,6-naphthyridine derivatives of the general formula I according to claim 1, characterised in that $R^1$ is an unsubstituted phenyl radical or a phenyl radical substituted by halogen, methyl, methoxy, nitro, cyano, trifluormethyl or difluoromethoxy or a thienyl, pyridyl or 2,1,3-benzooxadiazolyl radical; $R^2$ is a methyl, ethyl, n-propyl, isopropyl, n-butyl, sec.-butyl, isobutyl or benzyl radical; $R^3$ is hydrogen, a methyl, ethyl or isopropyl radical or a methoxycarbonyl, ethoxycarbonyl, propoxycarbonyl or isopropoxycarbonyl radical; $R^4$ is a methyl or ethyl radical and $R^5$ is a carboxyl or alkoxycarbonyl radical of the general formula III, wherein $R^7$ is either hydrogen, a methyl, ethyl, propyl, isopropyl, n-butyl, isobutyl, sec.-butyl, tert.-butyl, benzyl, 2-methylthioethyl, 2-methoxyethyl, 2-piperidinoethyl radical or an aminoalkyl radical of the general formula VI, wherein $R^9$ and $R^{10}$ may be the same or different and are hydrogen, a straight-chained or branched alkyl radical with 1 to 4 carbon atoms or a benzyl radical, and n is the number 2 or 3.

4. Process according to claim 1 or 2 for the preparation of 1,6 naphthyridine derivatives according to claim 1, characterised in tht $R^1$ is a phenyl, 2-chlorophenyl, 2-bromophenyl, 2-fluorophenyl, 2-nitrophenyl, 2-trifluoromethylphenyl, 2-cyanophenyl, 2-methylphenyl, 2-difluoromethoxyphenyl, 2-methoxyphenyl, 3-nitrophenyl, 2,3-dichlorophenyl, 2-chloro-6-fluorophenyl, 3-chloro-2-fluorophenyl, 2-thienyl, pyridyl or 2,1,3-benzoxadiazolyl radical; $R^2$ is a methyl, ethyl, n-propyl, isopropyl, n-butyl, sec-butyl, isobutyl or benzyl radical; $R^3$ is hydrogen, a methyl or ethoxycarbonyl radical; $R^4$ is a methyl or ethyl radical and $R^5$ is a methoxycarbonyl, ethoxycarbonyl, propoxycarbonyl, isopropoxycarbonyl, butoxycarbonyl, isobutoxycarbonyl, sec.-butoxycarbonyl, tert.-butoxycarbonyl, benzyloxycarbonyl, (2-methoxyethyl)oxycarbonyl, (2-methylthioethyl)oxycarbonyl, (2-piperidinoethyl)oxycarbonyl, (2-(N-methyl-N-phenylamino)ethyl)-oxycarbonyl, (2-diethylaminoethyl)oxycarbonyl, (2-aminoethyl)- oxycarbonyl, (2-dimethylaminoethyl)oxycarbonyl, (3-diethylaminopropyl)oxycarbonyl, (2-dibenzylaminoethyl)oxycarbonyl, (2-(N-benzyl-N-methylamino)ethyl)oxycarbonyl or (3-(N-benzyl-N-methylamino)propyl)oxycarbonyl radical.

5. Process according to claim 1 or 2 for the preparation of methyl (±)-1,4-dihydro-5-isopropoxy-2-methyl-4-(2-trifluoromethylphenyl)-1,6-naphthyridine-3-carboxylate and pharmacologically acceptable salts thereof and enantiomers thereof.

6. Process according to claim 1 or 2 for the preparation of 2-(N-benzyl-N-methylamino)-ethyl (±)-1,4-dihydro-5-isopropoxy-2-methyl-4-(2-trifluoromethylphenyl)-1,6-naphthyridine-3-carboxylate and pharmacologically acceptable salts thereof and enantiomers thereof.

7. Use of 1,6-naphthyridine derivatives according to claims 1 to 6 for the preparation of medicaments for combatting cardiac, cerebral and peripheral vascular diseases, ischaemiae, infarctions, thromboses, stenoses, arterial scleroses and for combatting cardiovascular diseases.

**Revendications pour les Etats contractants: BE, CH, DE, FR, GB, IT, LI, LU, NE, SE**

1. Dérivés de 1,6-naphtyridine de formule générale I:

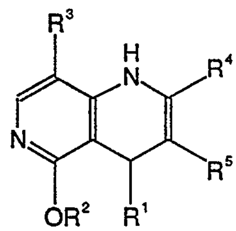

(I)

dans laquelle: $R^1$ représente un cycle phényl non substitué ou mono- ou disubstitué par des atomes d'halogène, des radicaux alkyl inférieur, alkoxy inférieur, alkylènedioxy inférieur, nitro, cyano, aminoalkyl inférieur, méthylthio, trifluorméthyl ou difluorométhoxy ou un cycle thiényl, pyridyl ou 2,1,3-benzoxadiazolyl; $R^2$ représente un groupe alkyl à chaîne droite ou ramifiée renfermant jusqu'à 4 atomes de carbone ou un groupe benzyl; $R^3$ représente un atome d'hydrogène ou un groupe alkyl à chaîne droite ou ramifiée renfermant jusqu'à 4 atomes de carbone dans le groupe alkyl; $R^4$ représente un radical alkyl à chaîne droite ou ramifiée renfermant jusqu'à 4 atomes de carbone; et $R^5$ représente un radical carboxyl ou un alkoxycarbonyl de formule générale III:

$$-CO_2R^7 \qquad (III)$$

dans laquelle: $R^7$ représente soit un atome d'hydrogène, un radical méthyl, éthyl, propyl, isopropyl, n-butyl, isobutyl, butyl secondaire, butyl tertiaire, benzyl, 2-pipéridinoéthyl, un radical alkoxyalkyl ou alkylthioalkyl de formule générale IV ou V:

$-(CH_2)_n-O-R^8$ (IV)

$-(CH_2)_n-S-R^8$ (V)

dans lesquelles: $R^8$ représente un groupe alkyl renfermant de 1 à 3 atomes de carbone et n est égal à 2 ou 3, ou un groupe aminoalkyl de formule générale VI:

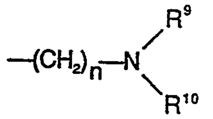

(VI)

dans laquelle: $R^9$ et $R^{10}$ peuvent être identiques ou différents et représentent un atome d'hydrogène, un groupe alkyl inférieur à chaîne droite ou ramifiée renfermant de 1 à 4 atomes de carbone ou un groupe benzyl; et n est égal à 2 ou 3, ainsi que le cas échéant, leurs sels pharmacologiquement compatibles.

2. Dérivés de 1,6-naphtyridine de formule générale I selon la revendication 1, caractérisés en ce que: $R^1$ représente un radical phényl non substitué ou substitué par un halogène, un radical méthyl, méthoxy, un groupe nitro, cyano, un radical trifluorométhyl ou difluorométhoxy ou un cycle thiényl, pyridyl ou 2,1,3-benzoxadiazolyl; $R^2$ représente un radical méthyl, éthyl, n-propyl, isopropyl, n-butyl, butyl secondaire, isobutyl, ou benzyl; $R^3$ représente un atome d'hydrogène, un radical méthyl, éthyl ou isopropyl ou un radical méthoxycarbonyl, éthoxycarbonyl, propoxycarbonyl ou isopropoxycarbonyl; $R^4$ représente un radical méthyl ou éthyl; et $R^5$ représente un radical carboxyl ou alkoxycarbonyl de formule générale III, dans laquelle: $R^7$ représente soit un atome d'hydrogène, soit un radical méthyl, éthyl, propyl, isopropyl, n-butyl, isobutyl, butyl secondaire, butyl tertiaire, benzyl, 2-méthylthioéthyl, 2-méthoxyéthyl, 2-pipéridinoéthyl ou un groupe aminoalkyl de formule générale VI, dans laquelle: $R^9$ et $R^{10}$ peuvent être identiques ou différents et représentent un atome d'hydrogène, un groupe alkyl inférieur à chaîne droite ou ramifiée renfermant de 1 à 4 atomes de carbone ou un groupe benzyl; et n est égal à 2 ou 3.

3. Dérivés de 1,6-naphtyridine selon la revendication 1, caractérisés en ce que: $R^1$ représente un radical phényl, 2-chlorophényl, 2-bromophényl, 2-nitrophényl, 2-trifluorométhylphényl, 2-cyanophényl, 2-phénylphényl, 2-trifluorométhoxyphényl, 2-méthoxyphényl, 3-nitrophényl, 2,3-dichlorophényl, 2-chloro-6-fluorophényl, 3-chloro-2-fluorophényl, 2-thiényl, pyridyl, ou 2,1,3-benzoxadiazolyl; $R^2$ représente un radical méthyl, éthyl, n-propyl, isopropyl, n-butyl, butyl secondaire, isobutyl, ou benzyl; $R^3$ représente un atome d'hydrogène, un radical méthyl ou éthoxycarbonyl; $R^4$ représente un radical méthyl ou éthyl; et $R^5$ représente un radical méthoxycarboxyl, éthoxycarbonyl, propoxycarbonyl, isopropoxycarbonyl, butoxycarbonyl, isobutoxycarbonyl, butoxycarbonyl secondaire, butoxycarbonyl tertiaire, benzyloxycarbonyl, 2-méthoxyéthyloxycarbonyl, 2-méthylthioéthylcarbonyl, 2-pipéridinoéthyloxycarbonyl, (2-(N-méthyl-N-phénylamino)éthyl)oxycarbonyl, (2-diéthylaminoéthyl)oxycarbonyl, (2-aminoéthyl)oxycarbonyl, (2-diméthylaminoéthyl)oxycarbonyl, (3-diméthylaminopropyl)oxycarbonyl, (2-dibenzylaminoéthyl)oxycarbonyl, (2-(N-benzyl-N-méthylamino)éthyl)-oxycarbonyl ou (3-(N-benzyl-N-méthylamino)propyloxycarbonyl.

4. Ethylester de l'acide (±)-1,4-dihydro-5-isopropoxy-2-méthyl-4-(2-trifluorométhylphényl)-1,6-naphtyridine-3-carboxylique.

5. [2-(N-benzyl-N-méthylamino)-éthyl]ester de l'acide (±)-1,4-dihydro-5-isopropoxy-2-méthyl- 4-(3-nitrophényl)-1,6-naphtyridine-3-carboxylique et ses sels pharmacologiquement compatibles.

6. Dérivés selon la revendication 1 à 5 sous la forme de leurs énantiomères.

7. Procédé de préparation de dérivés de 1,6-naphtyridine de formule générale I, selon la revendication 1 à 6, caractérisé en ce que l'on alkyle, d'une façon connue en soi, des dérivés de 1,6-naphtyridinone de formule générale VII:

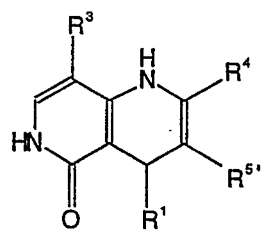

(VII)

dans laquelle: $R^1$, $R^3$ et $R^4$ ont la signification indiquée ci-dessus; et $R^5$ à la signification indiquée à l'exception d'un groupe carboxylique, en dérivés de formule générale I dans laquelle $R^1$, $R^3$ et $R^4$ ont la signification indiquée ci-dessus et $R^5$ à la signification indiquée à l'exception d'un groupe carboxylique, et on les convertit, le cas échéant, en leurs sels pharmacologiquement compatibles.

8. Procédé de préparation de dérivés de 1,6-naphtyridine de formule générale I, selon les revendications 1 à 5, dans lesquelles: $R^1$, $R^3$ et $R^4$ ont la signification indiquée ci-dessus; et $R^5$ représente un groupe carboxylique, caractérisé en ce que l'on procède d'une façon connue en soi à une dissociation hydrogénolytique de dérivés de 1,6-naphtyridinone de formule générale I dans laquelle $R^1$, $R^3$ et $R^4$ ont la signification indiquée ci-dessus; et $R^5$ représente un radical benzyloxycarbonyl; et, le cas échéant, on les convertit en leurs sels pharmacologiquement compatibles.

9. Médicament contenant, comme substance active, au moins un dérivé de 1,6-naphtyridine selon les revendications 1 à 6.

10. Utilisation de dérivés de 1,6-naphtyridine selon les revendications 1 à 6, pour la préparation de médicaments destinés à la lutte contre les maladies cardiaques, cérébrales et vasculaires périphériques, les ischémies, les infarctus, les thromboses, les sténoses, les artérioscléroses et à la lutte contre les maladies circulatoires.

**Revendications pour l'Etat contractant: AT**

1. Procédé de préparation de 1,6-naphtyridine de formule générale I:

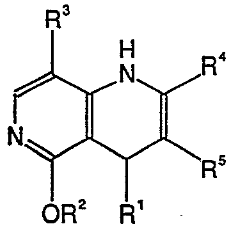

(I).

dans laquelle: $R^1$ représente un cycle phényl non substitué ou mono- ou disubstitué par des atomes d'halogène, des radicaux alkyl inférieur, alkoxy inférieur, alkylènedioxy inférieur, nitro, cyano, aminoalkyl inférieur, méthylthio, trifluorométhyl ou difluorométhoxy ou un cycle thiényl, pyridyl ou 2,1,3-benzoxadiazolyl; $R^2$ représente un groupe alkyl à chaîne droite ou ramifiée renfermant jusqu'à 4 atomes de carbone ou un groupe benzyl; $R^3$ représente un atome d'hydrogène ou un groupe alkyl à chaîne droite ou ramifiée renfermant jusqu'à 4 atomes de carbone dans le groupe alkyl; $R^4$ représente un radical alkyl à chaîne droite ou ramifiée renfermant jusqu'à 4 atomes de carbone; et $R^5$ représente un radical carboxyl ou alkoxycarbonyl de formula générale III:

$$-CO_2R^7 \quad (III)$$

dans laquelle: $R^7$ représente soit un atome d'hydrogène, un radical méthyl, éthyl, propyl, isopropyl, n-butyl, isobutyl, butyl secondaire, butyl tertiaire, benzyl, 2-pipéridinoéthyl, un radical alkoxyalkyl ou alkylthioalkyl de formule générales IV et V:

$$-(CH_2)_n-O-R^8 \quad (IV)$$
$$-(CH_2)_n-S-R^8 \quad (V)$$

dans lesquelles: $R^8$ représente un groupe alkyl renfermant de 1 à 3 atomes de carbone et n est égal à 2 ou 3, sous la forme de racémates ou sous la forme de leurs énantiomères ainsi que, le cas échéant, leurs sels pharmacologiquement compatibles, caractérisé en ce que l'on alkyle, de façon connue en soi, des 1,6-naphtyridinone de formule générale VII:

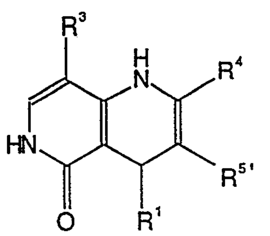

(VII)

dans laquelle: $R^1$, $R^3$ et $R^4$ ont la signification indiquée ci-dessus; et $R^5$ a la signification indiquée à l'exception d'un groupe carboxylique, en dérivés de formule générale I dans laquelle $R^1$, $R^3$ et $R^4$ ont la signification indiquée ci-dessus et $R^5$ a la signification indiquée à l'exception d'un groupe carboxylique, et on les convertit, le cas échéant, en leurs sels pharmacologiquement compatibles.

2. Procédé de préparation de dérivés de 1,6-naphtyridine de formule générale I selon la revendication 1, dans laquelle: $R^1$, $R^3$ et $R^4$ ont la signification indiquée ci-dessus; et $R^5$ représente un groupe carboxyl, caractérisé en ce que l'on procède de façon connue en soi à une dissociation hydrogénolytique de dérivés de 1-6, naphtyridinone de formule générale I dans laquelle: $R^1$, $R^3$ et $R^4$ ont la signification indiquée ci-dessus; et $R^5$ représente un radical benzyloxycarbonyl; et on les convertit, le cas échéant, en leurs sels pharmacologiquement compatibles.

3. Procédé selon la revendication 1 ou 2 de préparation de dérivés de 1,6-naphtyridine de formule générale I selon la revendication 1 ou 2, caractérisé en ce que: $R^1$ représente un radical phényl non substitué ou substitué par un halogène, un radical méthyl, méthoxy, un groupe nitro, cyano, un radical trifluorométhyl ou difluorométhoxy ou un cycle thiényl, pyridyl ou 2,1,3-benzoxadiazolyl; $R^2$ représente un radical méthyl, éthyl, n-propyl, isopropyl, n-butyl, butyl secondaire, isobutyl, ou benzyl; $R^3$ représente un atome d'hydrogène, un radical méthyl, éthyl ou isopropyl ou un radical méthoxycarbonyl, éthoxycarbonyl, propoxycarbonyl ou isopropoxycarbonyl; $R^4$ représente un radical méthyl ou éthyl; et $R^5$ représente un radical carboxyl ou alkoxycarbonyl de formule générale III, dans laquelle: $R^7$ représente soit un atome d'hydrogène, soit un radical méthyl, éthyl, propyl, isopropyl, n-butyl, isobutyl, butyl secondaire, butyl tertiaire, benzyl, 2-méthylthioéthyl, 2-méthoxyéthyl, 2-pipéridinoéthyl ou un groupe aminoalkyl de formule générale VI, dans laquelle: $R^9$ et $R^{10}$ peuvent être identiques ou différents et représentent un atome d'hydrogène, un groupe alkyl inférieur à chaîne droite ou ramifiée renfermant de 1 à 4 atomes de carbone ou un groupe benzyl; et n est égal à 2 ou 3.

4. Procédé selon la revendication 1 ou 2 de préparation de dérivés de 1,6-naphtyridine de formule générale I selon la revendication 1 ou 2, caractérisé en ce que: $R^1$ représente un radical phényl, 2-chlorophényl, 2-bromophényl, 2-nitrophényl, 2-trifluorométhylphényl, 2-cyanophényl, 2-phénylphényl, 2-trifluorométhoxyphényl, 2-méthoxyphényl, 3-nitrophényl, 2,3-dichlorophényl, 2-chloro-6-fluorophényl, 3-chloro-2-fluorophényl, 2-thiényl, pyridyl, ou 2,1,3-benzoxadiazolyl; $R^2$ représente un radical méthyl, éthyl, n-propyl, isopropyl, n-butyl, butyl secondaire, isobutyl, ou benzyl; $R^3$ représente un atome d'hydrogène, un radical méthyl ou éthoxycarbonyl; $R^4$ représente un radical méthyl ou éthyl; et $R^5$ représente un radical méthoxycarboxyl, éthoxycarbonyl, propoxycarbonyl, isopropoxycarbonyl, butoxycarbonyl, isobutoxycarbonyl, butoxycarbonyl secondaire, butoxycarbonyl tertiaire, benzyloxycarbonyl, 2-méthoxyéthyloxycarbonyl, 2-méthylthioéthylcarbonyl, 2-pipéridinoéthyloxycarbonyl, (2-(N-méthyl-N-phénylamino)éthyl)oxycarbonyl, (2-diéthylaminoéthyl)oxycarbonyl, (2-diméthylaminoéthyl)oxycarbonyl, (3-diméthylaminopropyl)oxycarbonyl, (2-dibenzylaminoéthyl)oxycarbonyl, (2-(N-benzyl-N-méthylamino)éthyl)oxycarbonyl ou (3-(N-benzyl-N-méthylamino)propyloxycarbonyl.

5. Procédé selon la revendication 1 ou 2 de préparation d'éthylester de l'acide (±)-1,4-dihydro-5-isopropoxy-2-méthyl-4-(2-trifluorométhylphényl)-1,6-naphtyridine-3-carboxylique et de ses sels et énantiomères pharmacologiquement compatibles.

6. Procédé selon la revendication 1 ou 2 de préparation de [2-(N-benzyl-N-méthylamino)-éthyl] ester de l'acide (±)-1,4-dihydro-5-isopropoxy-2-méthyl-4-(3-nitrophényl)-1,6-naphtyridine-3-carboxylique et de ses sels et énantiomères pharmacologiquement compatibles.

7. Utilisation de dérivés de 1,6-naphtyridine selon la revendication 1 à 6 pour la préparation de médicaments destinés à la lutte contre les maladies cardiaques, cérébrales et vasculaires périphériques, les ischémies, les infarctus, les thromboses, les sténoses, les artérioscléroses et de la lutte contre les maladies circulatoires.